# EUROPÄISCHE PATENTANMELDUNG

(11) **EP 4 420 608 A1**
(43) Veröffentlichungstag der Anmeldung: **28.08.2024**
(21) Anmeldenummer: 24158558.7
(22) Anmeldetag: 20.02.2024
(51) Int. Cl.: A61B 5/055, A61B 5/00, G01R 33/12, G01R 33/28

(54) **VORRICHTUNG UND VERFAHREN ZUR BESTIMMUNG EINER ANTWORT VON MAGNETISCHEN PARTIKELN AUF EIN ANREGUNGSMAGNETFELD**

(30) Priorität: 21.02.2023 DE 102023201537
(71) Anmelder: Fraunhofer-Gesellschaft zur Förderung der angewandten Forschung e.V., 80686 München (DE)
(72) Erfinder: Schumacher, Jonas, 23560 Lübeck (DE); Gräser, Matthias, 23568 Lübeck (DE)
(74) Vertreter: Altmann Stößel Dick Patentanwälte PartG mbB

(57) **Zusammenfassung**

Die Erfindung betrifft eine Vorrichtung (110) und ein Verfahren zur Bestimmung einer Antwort von magnetischen Partikeln (112) auf ein Anregungsmagnetfeld (114). Die Vorrichtung (110) umfasst:
- mindestens eine Sendeeinrichtung (122), die zur Erzeugung eines Anregungsmagnetfeldes (114) zur Anregung von magnetischen Partikeln (114) eingerichtet ist;
- mindestens eine Empfangseinrichtung (132), die zur Aufnahme einer Antwort der magnetischen Partikel (112) auf das Anregungsmagnetfeld (114) eingerichtet ist;
- mindestens eine Nachweiseinrichtung (142), die zur Aufnahme mindestens eines Wertes des Anregungsmagnetfeldes (114) oder von Störfeldern (144) eingerichtet ist;
- mindestens eine Signaltrennungseinrichtung (136, 136'), die dazu eingerichtet ist, um aus einem von der mindestens einen Empfangseinrichtung (132) erzeugten ersten Messsignal (134) mindestens ein Signal zu extrahieren; und
- eine Steuerungseinrichtung (140), die zur Ansteuerung der mindestens einen Sendeeinrichtung (122), der mindestens einen Empfangseinrichtung (132) und der mindestens einen Nachweiseinrichtung (142) sowie zur Bestimmung der von der mindestens einen Empfangseinrichtung (132) aufgenommenen Antwort der magnetischen Partikel (112) auf das Anregungsmagnetfeld (114) unter Berücksichtigung des mindestens einen Wertes des Anregungsmagnetfeldes (114) oder der Störfelder (144) eingerichtet ist.

Die Vorrichtung (110) und das Verfahren ermöglichen es, bei der Bestimmung der Antwort der magnetischen Partikel (112) auf ein Anregungsmagnetfeld (114) dynamische Veränderungen in dem von der Empfangseinrichtung (132) aufgenommenen ersten Messsignal (134) zu berücksichtigen. Hierdurch können geringe Partikelmengen detektiert werden und/oder vor allem im Hinblick auf Anwendungen an Personen geringere Anforderungen an die Sendekette (120) und/oder an eine Abschirmung gestellt werden.

## Beschreibung

### Gebiet der Erfindung

Die vorliegende Erfindung betrifft eine Vorrichtung und ein Verfahren zur Bestimmung einer Antwort von magnetischen Partikeln auf ein Anregungsmagnetfeld, insbesondere um *in vivo* eine räumliche Verteilung der magnetischen Partikel innerhalb eines Volumens zu bestimmen, wobei die magnetischen Partikel vorzugsweise verteilt in einem Fluid oder im Inneren eines Objekts, insbesondere in einem lebenden Patienten, vorliegen.

### Stand der Technik

Bei der Magnetpartikel-Bildgebung *(engl.* "Magnetic Particle Imaging", MPI) werden oszillierende Magnetfelder zur Anregung von magnetischen Partikeln, bevorzugt Nanopartikel, insbesondere super-paramagnetische Eisenoxid-Nanopartikel *(engl.* "super paramagnetic iron oxide nanoparticle", SPION), eingesetzt, um eine räumliche Verteilung der magnetischen Partikel *in vivo* zu bestimmen. Zur Eingrenzung auf ein festgelegtes Volumen kann das Anregungsmagnetfeld zusätzlich mit einem Selektionsmagnetfeld *(engl.* "selection field") überlagert werden. Mittels einer Empfangseinrichtung, die bevorzugt in Form mindestens einer Empfangsspule ausgestaltet sein kann, kann eine Änderung der Magnetisierung der magnetischen Partikel als Antwort der magnetischen Partikel auf das Anregungsmagnetfeld nachgewiesen werden. Allerdings interferiert in der Empfangseinrichtung ein von den Partikeln induziertes Messsignal sowohl mit dem Anregungsmagnetfeld als auch mit üblicherweise vorhandenen Störfeldern, die insbesondere von elektrischen Zuleitungen, aber auch von externen Quellen erzeugt werden können.

Zur Extraktion des Partikelsignals werden hierzu in der Regel analoge Band-Stopp-Filter verwendet, insbesondere um das Anregungsmagnetfeld zu unterdrücken, das eine um mehrere Größenordnungen höhere Amplitude als das Messsignal aufweisen kann. Weiterhin erfolgt üblicherweise ein Leermessungsabzug, wofür sowohl eine Messung mit Probe als auch zu einem anderen Zeitpunkt eine Messung ohne Probe durchgeführt wird und abschließend eine Korrektur der Messung mit Probe durch Subtraktion der Messung ohne Probe im Zeit- oder Frequenzbereich vorgenommen wird. Ferner existieren mehrere Vorschläge zur Erzielung einer Dämpfung des Anregungsmagnetfeldes in der Empfangseinrichtung.

Matthias Graeser, Tobias Knopp, Mandy Grüttner, Timo F. Sattel, und Thorsten M. Buzug, Analog receive signal processing for magnetic particle imaging, Med. Phys., 40: 042303, 2013 beschreiben Möglichkeiten, das in der Magnetpartikel-Bildgebung (MPI) auftretende Störsignal zu entfernen. Eine Möglichkeit besteht in einem Filterverfahren durch Unterdrückung des Anregungssignals mittels eines Bandstoppfilters. Hierbei werden jedoch Teile des gewünschten Partikelsignals entfernt, welche für die direkte Rekonstruktion der Partikelkonzentration erforderlich sind. Eine andere Möglichkeit, das gesamte Partikelsignal wiederherzustellen, besteht in der Auslöschung des Anregungssignals durch Einkopplung eines passenden Auslöschungssignals in die Empfangskette. Allerdings sind die Unterdrückungsraten, die in dem Auslöschungsverfahren erreicht werden können, nicht so hoch wie dem Filterverfahren, wodurch die Empfindlichkeit einschränkt wird. Um Vorteile beider Verfahren zu vereinen, wird vorgeschlagen, die Filter- und die Auslöschungsverfahren zu kombinieren.

Bo Zheng, Wisely Yang, Travis Massey, Patrick W. Goodwill und Steven M. Conolly, High-power active interference suppression in magnetic particle imaging, 2013 International Workshop on Magnetic Particle Imaging (IWMPI), 2013, pp. 1-1, beschreiben ein Verfahren zur aktiven Unterdrückung von Verzerrungsstörungen im erfassten MPI-Signal unter Verwendung einer transformatorgekoppelten Unterdrückungsschaltung.

Volkmar Schulz, Marcel Straub, Max Mahlke, Simon Hubertus, Twan Lammers und Fabian Kiessling, A field cancelation signal extraction method for magnetic particle imaging, IEEE Trans Magn. 2015; 51(2 Pt 1), schlagen analog zu Graeser et al. (2013) ein Konzept zur Feldauslöschung vor, bei dem eine Empfangsspule aus einer Reihenschaltung einer Primärspule in Kombination mit einer zusätzlichen Auslöschungsspule besteht. Die Geometrie der Primärspule wurde so gewählt, dass sie für das Signal der magnetischen Nanopartikel empfindlich ist, während die Auslöschungsspule so gewählt wurde, dass die gesamte induktive Einkopplung des Anregungsfeldes in die Empfangsspule minimiert wird. Durch eine Messung und Digitalisierung der Spannung über der Auslöschungsspule, vorzugsweise vorverarbeitet durch ein Tiefpassfilter und einen rauscharmen Verstärker, kann die Grundfrequenz des Anregungsfeldes numerisch kompensiert werden. Aufgrund des hohen Dynamikumfangs des Signals ist eine Detektion der im Sendesignal auftretenden unerwünschten Verzerrungen nur sehr begrenzt möglich.

Dennis Pantke, Nils Holle, Akshay Mogarkar, Marcel Straub und Volkmar Schulz, Multifrequency magneticparticle imaging enabled by α combined passive and active drive field feed-through compensation approach, Med. Phys. 46 (9), 2019, 4077-86, schlagen vor, um eine flexible Antriebsfeldfrequenz über eine große Bandbreite zu ermöglichen, einen kombinierten, passiven und aktiven Ansatz zur Kompensation eines einkoppelnden Anregungsfelds vor. Diese Kompensationstechnik ermöglicht außerdem den direkten Nachweis des Signals der super-paramagnetischen Eisenoxid-Nanopartikel bei der Grundanregungsfrequenz.

Hendrik Paysen, Olaf Kosch, James Wells, Norbert Loewa und Frank Wiekhorst, Characterization of noise and background signals in a magnetic particle imaging system, Phys. Med. Biol. 65 (2020) 235031, präsentieren eine umfassende Charakterisierung von Rausch- und Hintergrundsignalbeiträgen in Rohmessdaten, die von einem kommerziellen MPI-Scanner erfasst wurden. Hierzu wurde ein qualitatives Modell möglicher Signalquellen entwickelt und der quantitative Einfluss dieser Quellen anhand realer Messungen bestimmt. Diese Art der Charakterisierung kann nützliche Informationen für die Optimierung eines bestimmten MPI-Scanners liefern und darüber hinaus zur Umsetzung umfassender Verbesserungen der MPI-Technologie als Ganzes genutzt werden. Erstens erleichtert die quantitative Bewertung des Einflusses verschiedener Signalquellen die Einschätzung der Verbesserungsmöglichkeiten und eine Abschätzung aktueller und zukünftiger Empfindlichkeitsgrenzen. Zweitens bildet die Charakterisierung der zeitlichen Abhängigkeit von Rauschen und Hintergrundsignalen die Grundlage für anspruchsvollere Hintergrundkorrekturtechniken, einschließlich Software- und Hardwarelösungen.

M. Irfan, N. Dogan, T. Sapmaz, und A. Bingolbali, Development of MPI relaxometer for characterization of superparamagnetic nanoparticles, in Journal of Magnetism and Magnetic Materials 536, 2021, S.168082-1-8, beschreiben Design, Implementierung und experimentelle Messungen eines MPI-Relaxometers zur Charakterisierung superparamagnetischer Eisenoxid-Nanopartikel (SPIONs) als Tracer für Magnetic Particle Imaging (MPI). Das Relaxometer ist gegen elektromagnetische Interferenzen abgeschirmt und dient als MPI-Scanner mit Null-Magnetfeld bei 4,6 kHz und 9,9 kHz. Die Post-Processing-Analyse wurde an handelsüblichen Nanopartikeln durchgeführt, um Relaxationszeit, Auflösung, Frequenzspektrum der ungeraden Oberschwingungen bis zur 20. Oberschwingung und relative Signalstärke zu ermitteln, die für MPI wesentliche Parameter sind. Sinusförmige Anregungsmagnetfelder von 5 mT, 10 mT bzw. 15 mT wurden angelegt, um ihre Auswirkungen auf die Auflösung der Proben zu bewerten. Außerdem wurden Messungen bei zwei verschiedenen Frequenzen (4,6 kHz, 9,9 kHz) durchgeführt und die von der Antriebsfrequenz abhängige Relaxationszeit der magnetischen Nanopartikel berechnet.

Fabian Mohn, Tobias Knopp, Marija Boberg, Florian Thieben, Patryk Szwargulski und Matthias Graeser, System Matrix Based Reconstruction for Pulsed Sequences in Magnetic Particle lmaging, in: IEEE Transactions on Medical Imaging 41 (7), 2022, S. 1862-1873, beschreiben, dass eine Verbesserung von Auflösung und Empfindlichkeit die medizinischen Anwendungsmöglichkeiten der Magnetpartikel-Bildgebung erweitern wird. gemäß dem Stand der Technik wird eine sinusförmige Anregung verwendet, um superparamagnetische Nanopartikel in den nichtlinearen Teil ihrer Magnetisierungskurve zu bringen, um so ein Spektrum mit klarer Trennung von direkter Durchleitung und höheren Harmonischen, die durch Reaktion der Partikel verursacht werden, zu erhalten. Eine besondere Herausforderung ist die Unterscheidung von Partikel- und Anregungssignalen, die beide breitbandig sind. Eine weitere Herausforderung ist die Antriebsfeldsequenz selbst, da Teilchen, die sich nicht an gleichen räumlichen Position befinden, gleichzeitig reagieren können und nicht durch ihre Signalphase oder -form zu unterscheiden sind. Zur Überwindung eines möglichen Informationsverlusts bei der räumlichen Kodierung für hohe Amplituden, wird eine Überlagerung von Verschiebungs-Feldern und Rotation des Antriebsfelds vorgeschlagen. Dieser Ansatz ist in der Lage die Auflösung aufrechtzuerhalten, unabhängig von der Sequenz, wenn die Antwort auf gepulste Sequenzen noch Informationen in der Phase kodiert.

Florian Thieben, Tobias Knopp, Marija Boberg, Fynn Foerger, Matthias Graeser und Martin Möddel, On the Receive Path Calibration of Magnetic Particle Imaging Systems, IEEE Transactions on Instrumentation and Measurement, Vol. 72, 2023, 1000915, stellen ein allgemeines Mehrkanal-Kalibrierungsverfahren für induktive Empfangswege für Magnetpartikel-Bildgebung (MPI) vor. Das Kalibrierungsverfahren wurde verallgemeinert, um auch nicht-orthogonale und inhomogene Empfangsspulen berücksichtigen zu können. Die MPI-Übertragungsfunktionen von falsch ausgerichteten Empfangsspulen wurde verwendet, um deren überlagerte Empfangssignale vom Empfangspfad zu entkoppeln. Die Ergebnisse ermöglichen den Vergleich von MPI-Signalen aus verschiedenen Geräten und können zur Normalisierung von Messungen und Systemfunktionen in Geräten mit austauschbaren Empfangsspulen verwendet werden.

DE 10 2018 204 311 B3 offenbart ein Verfahren zur Magnetpartikelbildgebung, umfassend das zeitlich wiederholte Detektieren der magnetischen Antwort einer vorverabreichten räumlichen Verteilung magnetisierbarer Partikel in einem Messvolumen auf ein vorbestimmtes Anregungsmagnetfeld mit harmonischer Zeitabhängigkeit, wobei wenigstens ein feldfreier Punkt entlang einer vorbestimmten periodischen Trajektorie durch das Messvolumen bewegt wird; die detektierte magnetische Antwort zu jedem Erfassungszeitpunkt mit dem Ort des wenigstens einen feldfreien Punktes registriert wird; aus der detektierten magnetischen Antwort auf die Konzentration der Partikel am jeweiligen Ort des feldfreien Punktes geschlossen wird; wenigstens ein Voxelmodell für wenigstens ein Teilvolumen des Messvolumens erstellt wird, wobei den Voxeln Werte für die Konzentration der Partikel zugeordnet werden, die sich aus den entlang der Trajektorie des feldfreien Punktes bestimmten Konzentrationen durch Interpolation ergeben, wobei numerisch berechnete Schnitte durch das Voxelmodell als Bilder ausgegeben werden; wobei die magnetische Antwort in ein elektrisches Signal überführt und mit einem elektrischen Gegensignal näherungsweise kompensiert wird, wobei ein elektrisches Residualsignal gebildet wird, das Residualsignal digitalisiert wird, ein digitales Stellsignal auf Basis des digitalisierten Residualsignals berechnet wird, das digitale Stellsignal in das elektrische Gegensignal konvertiert wird und das digitale Stellsignal und das digitalisierte Residualsignal je einen Anteil der detektierten magnetischen Antwort bilden.

DE 10 2020 118 102 B4 offenbart ein Verfahren zur Erfassung der magnetischen Antwort einer Verteilung magnetisierbarer Partikel auf ein magnetisches Anregungsfeld mit einer vorbestimmten Grundfrequenz, wobei die magnetische Antwort in wenigstens einer Detektionsspule ein Spannungssignal mit Anteilen der Harmonischen der Grundfrequenz induziert und wobei in wenigstens einer Kompensationsspule ein vom magnetischen Anregungsfeld induziertes Kompensationssignal erzeugt und mit dem Spannungssignal destruktiv superponiert wird, so dass ein kompensiertes Spannungssignal an einem Ausgang der Spulenanordnung abgegriffen wird. Hierzu erfolgt ein Zuführen des kompensierten Spannungssignals zu einem ersten Paar von Übertragungsgliedern mit einem vorbestimmten Bandbreitenbereich um die führende Harmonische umfassend ein erstes Übertragungsglied und ein zweites Übertragungsglied; ein Isolieren der führenden Harmonischen mit dem ersten Übertragungsglied, aufweisend eine Band des isolierten Signalanteils; ein Unterdrücken der führenden Harmonischen mit dem zweiten Übertragungsglied, aufweisend eine Bandstopp- oder Hochpasscharakteristik und ein Abgreifen der verbleibenden Signalanteile; ein optionales Zuführen der verbleibenden Signalanteile zu einem weiteren Paar von Übertragungsgliedern mit einem vorbestimmten Bandbreitenbereich um die nächsthöhere Harmonische und wiederholen der beiden vorangehenden Schritte; ein Digitalisieren des verbleibenden Signals, wobei jedem Digitalisierungsschritt im jeweiligen Übertragungsglied eine Signalverstärkung derart vorgeschaltet wird, dass die vorbekannte Messdynamik der Digitalisierungseinrichtung ausgeschöpft wird; und eine Rekonstruktion der magnetischen Antwort der Partikelverteilung aus den digitalisierten Signalen unter Berücksichtigung der separaten Signalverstärkungen und vorab ermittelter Kalibrierdaten.

DE 10 2020 212 466 A1 offenbart ein Gerät und ein Verfahren zur Analyse einer wässrigen Probe auf das Vorhandensein eines Analyten, wobei die Probe mit für den Analyten spezifischen magnetischen Nanopartikeln versetzt ist. Das Gerät eignet sich zur Erfassung der magnetischen Antwort einer Probe mit magnetisierbaren Partikel auf ein magnetisches Anregungsfeld mit einer Grundfrequenz, wobei die magnetische Antwort in mindestens einer Detektionsspule ein Spannungssignal mit Anteilen von Harmonischen der Grundfrequenz induziert. Zur Verbesserung der Genauigkeit des Verfahrens wird analog zu Volkmar Schulz et al., 2015, s. o., und zu Matthias Graeser et al., 2013, s. o., eine Einrichtung zur Feldauslöschung vorgeschlagen, die in einer bevorzugten Ausgestaltung eine Empfangsspule aus einer Reihenschaltung einer Primärspule in Kombination mit einer zusätzlichen Auslöschungsspule umfasst. Sowohl in Volkmar Schulz et al., 2015, s. o., als auch in DE 10 2020 212 466 A1 wird dies mit der Messung des über der Auslöschungsspule anliegenden Spannungssignals kombiniert. Aufgrund eines hohen Dynamikumfangs des Signals ist eine Detektion der im Sendesignal auftretenden unerwünschten Verzerrungen nur sehr begrenzt möglich.

### Aufgabe der vorliegenden Erfindung

Ausgehend hiervon, besteht die Aufgabe der vorliegenden Erfindung darin, eine Vorrichtung und ein Verfahren zur Bestimmung einer Antwort von magnetischen Partikeln auf ein Anregungsmagnetfeld bereitzustellen, welche die aufgeführten Nachteile und Einschränkungen des Standes der Technik zumindest teilweise überwinden.

Insbesondere sollen es die vorgeschlagene Vorrichtung und das vorliegende Verfahren ermöglichen, bei der Bestimmung der Antwort der magnetischen Partikel auf ein Anregungsmagnetfeld dynamische Veränderungen in dem von der Empfangseinrichtung aufgenommenen Messsignal zu berücksichtigen, die in der Regel insbesondere in Folge von Änderungen des Anregungsmagnetfeldes aufgrund von Temperaturänderungen in der Sendekette als auch in Folge von üblicherweise vorhandenen Störfeldern, die von elektrischen Zuleitungen, aber auch von externen Quellen herrühren können, auftreten können.

### Offenbarung der Erfindung

Diese Aufgabe wird durch eine Vorrichtung und ein Verfahren zur Bestimmung einer Antwort von magnetischen Partikeln auf ein Anregungsmagnetfeld gemäß den Merkmalen der unabhängigen Ansprüche gelöst. Vorteilhafte Weiterbildungen, welche einzeln oder in beliebiger Kombination realisierbar sind, sind in den abhängigen Ansprüchen und in der nachfolgenden Beschreibung dargestellt.

In einem ersten Aspekt betrifft die vorliegende Erfindung eine Vorrichtung zur Bestimmung einer Antwort von magnetischen Partikeln auf ein Anregungsmagnetfeld. Die Vorrichtung umfasst hierbei
- mindestens eine Sendeeinrichtung, die zur Erzeugung eines Anregungsmagnetfeldes zur Anregung von magnetischen Partikeln eingerichtet ist;
- mindestens eine Empfangseinrichtung, die zur Aufnahme einer Antwort der magnetischen Partikel auf das Anregungsmagnetfeld eingerichtet ist;
- mindestens eine Nachweiseinrichtung, die zur Aufnahme mindestens eines Wertes des Anregungsmagnetfeldes oder von Störfeldern eingerichtet ist;
- mindestens eine Signaltrennungseinrichtung, die dazu eingerichtet ist, um aus einem von der mindestens einen Empfangseinrichtung erzeugten ersten Messsignal mindestens ein Signal zu extrahieren; und
- eine Steuerungseinrichtung, die
   o zur Ansteuerung der mindestens einen Sendeeinrichtung,
   o zur Ansteuerung der mindestens einen Empfangseinrichtung,
   o zur Ansteuerung der mindestens einen Nachweiseinrichtung,
   o zur Bestimmung der von der mindestens einen Empfangseinrichtung aufgenommenen Antwort der magnetischen Partikel auf das Anregungsmagnetfeld, und
   o zur Berücksichtigung des mindestens einen Wertes des Anregungsmagnetfeldes und der Störfelder bei der Bestimmung der Antwort der magnetischen Partikel auf das Anregungsmagnetfeld eingerichtet ist.

Die vorliegende Vorrichtung zur Bestimmung einer Antwort von magnetischen Partikeln auf ein Anregungsmagnetfeld kann einteilig oder mehrteilig aufgebaut sein. Insbesondere können eine die Sendeeinrichtung umfassende Sendekette, eine die Empfangseinrichtung umfassende Empfangskette, die mindestens eine Nachweiseinrichtung, die mindestens eine Signaltrennungseinrichtung und die Steuerungseinrichtung als gesonderte Komponenten ausgestaltet sein.

Die vorliegende Vorrichtung ist zur Bestimmung einer Antwort von magnetischen Partikeln auf ein Anregungsmagnetfeld eingerichtet. Der Begriff der "Bestimmung" bezeichnet einen Vorgang zur Ermittlung mindestens eines repräsentativen Wertes aus einem Messsignal, der in mindestens seinem Messschritt aufgenommen wurde, in einem anschließenden Auswertungsschritt. Der Begriff "Antwort" bezeichnet eine Reaktion der magnetischen Partikel auf das Anregungsmagnetfeld, wodurch eine lokale Änderung des Anregungsmagnetfeldes oder der Magnetisierung der Partikel ausgelöst durch die magnetischen Partikel, wobei die Änderung des Anregungsmagnetfeldes oder der Magnetisierung der Partikel als Messsignal aufgenommen werden kann. Bei den magnetischen Partikeln handelt es sich bevorzugt um Nanopartikel, insbesondere um super-paramagnetische Eisenoxid-Nanopartikel *(engl.* "su-perparamagnetic iron oxide nanoparticle", SPION); eine Verwendung mindestens einer anderen Art von magnetischen Partikeln ist jedoch möglich. Der Begriff des "Anregungsmagnetfeldes" bezeichnet ein magnetisches Feld, das derart ausgestaltet ist, dass hierdurch eine Anregung der magnetischen Partikel, die durch das Anregungsmagnetfeld beaufschlagt werden, erfolgen kann. Hierbei umfasst das Anregungsmagnetfeld mindestens eine Grundfrequenz, die von einer Sendeeinrichtung, die zur Erzeugung des Anregungsmagnetfeldes zur Anregung der magnetischen Partikel eingerichtet ist, erzeugt wird. In einer bevorzugten Ausgestaltung kann das Anregungsmagnetfeld, insbesondere zur Eingrenzung der Anregung auf ein festgelegtes Volumen, zusätzlich mit einem Selektionsmagnetfeld *(engl.* "selection field"), das vorzugsweise als Gradientenfeld ausgestaltet sein kann, überlagert werden.

Die vorliegende Vorrichtung umfasst mindestens eine Sendeeinrichtung. Der Begriff der "Sendeeinrichtung" bezeichnet hierbei eine Einrichtung, die zur Erzeugung eines Anregungsmagnetfeldes zur Anregung von magnetischen Partikeln eingerichtet ist. Die Sendeeinrichtung kann vorzugsweise als Sendespule *(engl.* "transmit coil") ausgestaltet sein, die das gewünschte Anregungsmagnetfeld mittels Beaufschlagung durch einen elektrischen Strom erzeugt. Andere Ausgestaltungen der Sendeeinrichtung sind jedoch möglich. Die Sendeeinrichtung kann vorzugsweise Teil einer Sendekette sein. Der Begriff der "Sendekette" bezeichnet eine Hintereinanderschaltung von mindestens zwei gesonderten elektronischen Komponenten, die zur Erzeugung des gewünschten Anregungsmagnetfeldes beitragen können. Vorzugsweise kann die Sendekette zusätzlich zur Sendeeinrichtung mindestens einen Leistungsverstärker (*engl.* "power amplifier") und/oder mindestens einen Bandpassfilter *(engl.* "band pass filter") und/oder mindestens eine Einrichtung zur Impedanz-Anpassung *(engl.* "impedance matching device") umfassen, welche der Sendeeinrichtung derart vorgeschaltet sind, dass ein die Sendeeinrichtung beaufschlagender Strom zuvor die mindestens eine weitere elektronische Komponente in der Sendekette durchläuft, bevor die Sendeeinrichtung damit beaufschlagt wird.

Der Begriff des "Leistungsverstärkers" bezeichnet hierbei eine Einrichtung, die ein, insbesondere aus der Steuerungseinrichtung, eingehendes elektronisches Signal derart verarbeitet, dass eine elektrische Leistung eines ausgehenden elektronischen Signals, das bevorzugt an das Bandpassfilter ausgegeben wird, die elektrische Leistung des eingehenden elektronischen Signals übersteigt, vorzugsweise deutlich übersteigt, wobei die Differenz der beiden elektrischen Leistungen einer Energiequelle entnommen wird. Die Verstärkung des Leistungsverstärkers kann bevorzugt derart eingestellt sein, dass die Leistung des ausgehenden elektronischen Signals ungefähr der vorgesehenen Leistung des Anregungsmagnetfelds entspricht. Der Begriff des "Bandpassfilters" bezeichnet einen Filter, der dazu eingerichtet ist, Frequenzen aus einem festlegbaren, als "Band" bezeichneten Frequenzbereich durchzulassen, während außerhalb des Bandes liegende Frequenzen einer hohen Dämpfung unterworfen werden. Das Bandpassfilter kann hierbei vorzugsweise derart eingerichtet sein, dass diejenigen Frequenzen durchgelassen werden, die zur Erzeugung des Anregungsmagnetfeldes vorgesehen sind. Der Begriff "Impedanz-Anpassung" bezeichnet ein Vorgehen zur Anpassung einer Impedanz eines Eingangs einer Last, hier der Sendeeinrichtung, an die Impedanz einer Quelle, hier eines Ausgangs der Steuerungseinrichtung oder, bevorzugt, des Leistungsverstärkers. Eine andere Ausgestaltung der Sendekette und/oder eine Einbeziehung von anderen oder weiteren Komponenten in die Sendekette sind möglich. Insbesondere kann die Sendekette weiterhin eine Einrichtung zur Erzeugung eines Selektionsmagnetfeldes umfassen; die Einrichtung zur Erzeugung eines Selektionsmagnetfeldes kann jedoch auch unabhängig von der Sendekette ausgestaltet sein.

Die vorliegende Vorrichtung umfasst mindestens eine Empfangseinrichtung. Der Begriff der "Empfangseinrichtung" bezeichnet hierbei eine Einrichtung, die zur Aufnahme einer Antwort der magnetischen Partikel auf das Anregungsmagnetfeld eingerichtet ist. Der Begriff der "Aufnahme" bezeichnet einen Vorgang, mittels welchem mindestens ein Messsignal, bevorzugt in Form eines elektronischen oder optischen Messsignals, erzeugt wird. Die Empfangseinrichtung kann vorzugsweise als Empfangsspule *(engl.* "receive coil") ausgestaltet sein, in welcher das Anregungsmagnetfeld mittels Beaufschlagung einen elektrischen Strom induziert, der als Messsignal dient. Andere Ausgestaltungen der Empfangseinrichtung sind jedoch möglich. Die Empfangseinrichtung kann vorzugsweise Teil einer Empfangskette sein. Der Begriff der "Empfangskette" bezeichnet eine Hintereinanderschaltung von mindestens zwei gesonderten elektronischen Komponenten, die zur Aufnahme der Antwort der magnetischen Partikel auf das Anregungsmagnetfeld beitragen können. Die Empfangskette umfasst zusätzlich zur Empfangseinrichtung mindestens eine Signaltrennungseinrichtung *(engl.* "signal separation device") und kann zusätzlich einen rauscharmen Verstärker *(engl.* "low-noise amplifier") umfassen, welche der Empfangseinrichtung derart nachgeschaltet sind, dass ein von der Empfangseinrichtung erzeugtes Messsignal anschließend die mindestens eine weitere elektronische Komponente in der Empfangskette durchläuft, bevor es zur Steuerungseinrichtung gelangt.

Wie oben erwähnt, ist die mindestens eine Signaltrennungseinrichtung dazu eingerichtet, um aus einem von der mindestens einen Empfangseinrichtung erzeugten ersten Messsignal mindestens ein Signal zu extrahieren. In einer besonders bevorzugten Ausgestaltung kann die mindestens eine Signaltrennungseinrichtung zusätzlich dazu eingerichtet sein, um für das Extrahieren des mindestens eines Signals ferner ein von der mindestens einen Nachweiseinrichtung erzeugtes weiteres Messsignal zu verwenden. Der Begriff der "Signaltrennungseinrichtung" bezeichnet hierbei eine Einrichtung, die dazu eingerichtet ist, um aus einem Messsignal mindestens ein Signal zu extrahieren. Die Signaltrennungseinrichtung kann insbesondere zur Anpassung eines Dynamikumfangs eines in der Empfangseinrichtung erzeugten ersten Messsignals oder eines von der mindestens einen Nachweiseinrichtung erzeugten weiteren Messsignals eingerichtet sein. Durch eine Nachverarbeitung eines ersten Messsignals oder eines weiteren Messsignals in der mindestens einen Signaltrennungseinheit kann der Dynamikumfang des jeweiligen Messsignals angepasst werden. Dadurch kann eine präzisere Berücksichtigung von unerwünschten Einflüssen auf das jeweilige Messsignal ermöglicht werden. Die Signaltrennungseinrichtung kann bevorzugt als Bandstoppfilter ausgestaltet sein, wobei der Begriff des "Bandstoppfilters" bezeichnet einen Filter, der dazu eingerichtet ist, Frequenzen aus einem festlegbaren, als "Band" bezeichneten Frequenzbereich einer hohen Dämpfung zu unterwerfen, während außerhalb des Bandes liegende Frequenzen durchgelassen werden. Alternativ oder ergänzend kann die Signaltrennungseinrichtung auch das Konzept zur Auslöschung des Anregungssignals gemäß M. Gräser et al., 2013, s.o. umfassen. Das Bandstoppfilter ist hierbei vorzugsweise derart ausgestaltet, dass es diejenigen Frequenzen herausfiltert, die den Frequenzen des Anregungsmagnetfeldes entsprechen.

Der Begriff des "rauscharmen Verstärkers" bezeichnet hierbei eine Einrichtung, die ein, insbesondere aus dem der Empfangseinrichtung nachgeschalteten Bandstoppfilter, eingehendes elektronisches Signal derart verarbeitet, dass eine elektrische Leistung eines ausgehenden elektronischen Signals, das bevorzugt an die Steuerungseinrichtung ausgegeben wird, die elektrische Leistung des eingehenden elektronischen Signals dahingehend übersteigt, dass das ausgehende elektronische Signal ein hohes Signal-zu-Rausch-Verhältnis *(engl.* "signal-to-noise ratio", SNR) aufweist. Bei geeigneter Auslegung des rauscharmen Verstärkers kann ein bei fehlendem Partikelsignal aufgenommenes Hintergrundsignal mit höherer Amplitude als im Empfangssignal erzeugt werden kann. Dadurch kann der maximale Dynamikumfang ausgenutzt und das Partikelsignal sehr präzise extrahiert werden. Eine andere Ausgestaltung der Empfangskette und/oder eine Einbeziehung von anderen oder weiteren Komponenten in die Empfangskette sind möglich.

Die vorliegende Vorrichtung umfasst mindestens eine Nachweiseinrichtung. Der Begriff der "Nachweiseinrichtung" bezeichnet hierbei eine Einrichtung, die zur Aufnahme mindestens eines Wertes des Anregungsmagnetfeldes oder von Störfeldern eingerichtet ist. Der Begriff des "Störfeldes" bezeichnet ein weiteres magnetisches Feld, das ein, auch als "Hintergrundsignal" bezeichnetes Messsignal in der Nachweiseinrichtung erzeugen kann, wobei das in der Nachweiseinrichtung erzeugte Messsignal weder durch das Anregungsmagnetfeld noch durch die Antwort der magnetischen Partikel auf das Anregungsmagnetfeld erzeugt wird. Hierbei können die Störfelder auch von der mindestens einen Grundfrequenz abweichende Frequenzen des Anregungsmagnetfeldes, insbesondere höhere Harmonische der mindestens einen Grundfrequenz, umfassen. Als Quellen für das weitere magnetische Feld können einerseits elektrische Komponenten und Leitungen innerhalb der Vorrichtung und andererseits externe Quellen außerhalb der Vorrichtung, insbesondere von elektrische Komponenten und Leitungen, welche sich in der Nähe der Vorrichtung befinden, dienen. Die Nachweiseinrichtung kann somit insbesondere dazu verwendet werden, um zeitliche Änderungen des Anregungsmagnetfeldes aufgrund von Temperaturänderungen in der Sendekette, die insbesondere in Folge einer Erwärmung von elektrischen Komponenten und Leitungen in der Sendekette, typischerweise in der mindestens einen Sendeeinrichtung und dem mindestens einen Leistungsverstärker, auftreten können, und/oder ein Vorhandensein von Störfeldern nachzuweisen, welche bei der Bestimmung der Antwort der magnetischen Partikel auf das Anregungsmagnetfeld berücksichtigt werden können. Der Begriff der "Berücksichtigung" bezeichnet ein Vorgehen, bei welchem zur Bestimmung eines gesuchten Wertes aus einem ersten Messsignal ein zweites Messsignal mit einfließt.

In einer besonders bevorzugten Ausgestaltung kann die mindestens eine Nachweiseinrichtung dazu eingerichtet sein, dass die Aufnahme mindestens eines ersten Messsignals, welches der Antwort der magnetischen Partikel auf das Anregungsmagnetfeld entspricht, im gleichen Zeitraum erfolgt wie die Aufnahme mindestens eines zweiten Messsignals, welches dem mindestens einem Wert des Anregungsmagnetfeldes und der Störfelder, entspricht. Der Begriff des "gleichen Zeittraums" bezeichnet ein Zeitintervall, innerhalb dessen sowohl das mindestens eine erste Messsignal als auch das mindestens eine zweite Messsignal aufgenommen werden. Somit können sowohl das mindestens eine erste Messsignal als auch das mindestens eine zweite Messsignal Berücksichtigung bei der Bestimmung der Antwort der magnetischen Partikel auf das Anregungsmagnetfeld finden. Auf diese Weise lassen sich zeitliche Veränderungen in dem von der Empfangseinrichtung aufgenommenen Messsignal berücksichtigen, die einerseits auf dynamische Veränderungen in dem Anregungsmagnetfeld und/oder in der Antwort der magnetischen Partikel auf das Anregungsmagnetfeld zurückgehen können oder andererseits auf Störfelder innerhalb und/oder außerhalb der Vorrichtung beruhen können.

In einer bevorzugten Ausgestaltung kann die Nachweiseinrichtung mindestens eine zusätzliche Empfangseinrichtung umfassen. Bevorzugt kann die zusätzliche Empfangseinrichtung zumindest eine zusätzliche Empfangsspule aufweisen, vorzugsweise ein weiteres Exemplar der von der Empfangseinrichtung umfassten Empfangsspule. Während die von der Empfangseinrichtung umfasste Empfangsspule zur Bestimmung der Antwort der magnetischen Partikel auf das Anregungsmagnetfeld eingerichtet ist, ist die zusätzliche Empfangsspule bevorzugt derart angeordnet, dass hierdurch keine Bestimmung der Antwort der magnetischen Partikel auf das Anregungsmagnetfeld erfolgen kann. Auf diese Weise kann die zusätzliche Empfangsspule bevorzugt dazu dienen, mindestens einen Wert des Anregungsmagnetfeldes und/oder der Störfelder aufzunehmen.

In einer besonderen Ausgestaltung kann die zusätzliche Empfangseinrichtung derart angeordnet sein, dass eine destruktive Überlagerung zur Empfangseinrichtung erfolgt, die zur Bestimmung der Antwort der magnetischen Partikel auf das Anregungsmagnetfeld eingerichtet ist. Der Begriff der "destruktiven Überlagerung" bezeichnet eine elektrische Verschaltung von mindestens zwei Einrichtungen auf einer Weise, dass sich die von der jeweiligen Einrichtung erzeugten Signale gegenseitig auslöschen. In einer besonderen Ausgestaltung kann die zusätzliche Empfangseinrichtung derart angeordnet sein, dass eine destruktive Überlagerung zur Empfangseinrichtung erfolgt wodurch sich die von der jeweiligen Einrichtung erzeugten Messsignale dann gegenseitig auslöschen, wenn keine magnetischen Partikel in dem Volumen, das von dem Anregungsmagnetfeld beaufschlagt wird, vorhanden sind. Wird die zusätzliche Empfangseinrichtung jedoch derart angeordnet, dass zusätzlich zu dem Anregungsmagnetfeld und der Antwort der magnetischen Partikel auf das Anregungsmagnetfeld zusätzlich auch noch die Störfelder ein Messsignal erzeugen können, so kann die zusätzliche Empfangseinrichtung in dieser Ausgestaltung das Hintergrundsignal, das von den Störfeldern erzeugt wird, direkt als Messsignal bereitstellen.

In einer weiteren besonderen Ausgestaltung kann die Vorrichtung zudem mindestens eine zusätzliche Sendeeinrichtung umfassen. In dieser Ausgestaltung kann die mindestens eine zusätzliche Sendeeinrichtung vorzugsweise in Serie mit der mindestens einen Sendeeinrichtung, die zur Erzeugung eines Anregungsmagnetfeldes zur Anregung von magnetischen Partikeln eingerichtet ist, geschaltet sein. Bevorzugt kann die zusätzliche Sendeeinrichtung zumindest eine zusätzliche Sendespule aufweisen, vorzugsweise ein weiteres Exemplar der von der Sendeeinrichtung umfassten Sendespule. Vorzugsweise kann die zusätzliche Sendespule derart angeordnet sein, dass hierdurch keine Anregung der magnetischen Partikel erfolgen kann. Auf diese Weise kann die zusätzliche Sendespule dazu dienen, ein Magnetfeld mit denselben Eigenschaften wie das Anregungsmagnetfeld zu erzeugen, welches vorzugsweise von einer oben oder unten beschriebenen zusätzlichen Empfangsspule erfasst werden kann.

In einer besonderen Ausgestaltung kann die zusätzliche Sendeeinrichtung derart mit der zusätzliche einen Sendeeinrichtung verschaltet sein, dass sich hierdurch eine Auslöschungseinrichtung ergibt, in welcher eine destruktive Überlagerung in der zugeordneten Empfangseinrichtung erfolgt, die zur Bestimmung der Antwort der magnetischen Partikel auf das Anregungsmagnetfeld eingerichtet ist. Dies kann sowohl durch eine jeweils entgegengesetzte Wickel- und/oder Verschaltungsrichtung auf der Sendeseite und/oder der Empfangsseite erfolgen. Auf diese Weise kann über die nicht zur Bestimmung der Antwort der magnetischen Partikel auf das Anregungsmagnetfeld eingerichteten Empfangseinheit der Auslöschungseinheit das Hintergrundsignal, das von den Störfeldern erzeugt wird, als Messsignal bereitgestellt werden. In einer besonderen Ausgestaltung ist dies auch durch Hinzufügen einer zusätzlichen Empfangseinheit im nicht zur Bestimmung der Antwort der magnetischen Partikel auf das Anregungsmagnetfeld eingerichteten Teil der Auslöschungseinheit möglich.

In einer weiteren bevorzugten Ausgestaltung kann die Nachweiseinrichtung mindestens eine Einrichtung zum Nachweis eines elektrischen Stroms, insbesondere eine Rogowskispule, umfassen. Die Begriffe "Rogowskispule" oder "Luftspule" bezeichnen eine elektromagnetische Spule, die zwar einen elektrisch leitende Leiterdraht, jedoch keinen ferromagnetischen Kern aufweist, wobei der Leiterdraht um einen festen Körper gewickelt sein kann, welcher elektrisch nicht-leitend und nicht-ferromagnetisch ist. Zur Messung eines elektrischen Wechselstroms in einem stromführenden Leiter, wird die Rogowskispule um diesen stromführenden Leiter gelegt, wodurch ein veränderliches Magnetfeld erzeugt wird, das zwischen den Enden des Leiterdrahts der Rogowskispule zu einem Spannungsabfall führt, welcher als Messsignal der Rogowskispule dienen kann. Die Rogowskispule kann grundsätzlich an einer beliebigen Stelle in der Sendekette angeordnet sein, bevorzugt ist jedoch eine Anordnung in unmittelbarer Nähe der mindestens einen Sendeeinrichtung. Auf diese Weise kann ein Strom in der Sendekette erfasst werden, der sich zur Bestimmung des Wertes des die magnetischen Partikel anregenden Magnetfelds eignet. In einer weiteren Ausgestaltung kann die Rogowskispule auch durch eine Spule mit einem Kern aus einem magnetisierbaren Material ersetzt werden. Die Positionierung kann dabei analog zur Rogowskispule an einer beliebigen Stelle in der Sendekette angeordnet sein, bevorzugt ist jedoch eine Anordnung in unmittelbarer Nähe der mindestens einen Sendeeinrichtung.

In einer weiteren bevorzugten Ausgestaltung kann die Nachweiseinrichtung mindestens einen Magnetfeldsensor umfassen. Als Magnetfeldsensor kann vorzugsweise eine Hall-Sonde oder ein XMR-Sensor dienen, die sich aufgrund ihres kompakten Aufbaus besonders für den vorliegenden Zweck eignen; die Verwendung eines anderen Magnetfeldsensors ist jedoch möglich. Der mindestens einen Magnetfeldsensor kann innerhalb des Anregungsmagnetfeldes, jedoch außerhalb des Volumens angeordnet sein, in welchem die magnetischen Partikel vorliegen. In einer besonders bevorzugten Ausgestaltung kann der mindestens einen Magnetfeldsensor außerhalb des Anregungsmagnetfeldes angeordnet sein, insbesondere um den mindestens einen Magnetfeldsensor dazu einzusetzen, um Störfelder, die von externe Quellen außerhalb der Vorrichtung, insbesondere von elektrische Komponenten und Leitungen, welche sich in der Nähe der Vorrichtung befinden, hervorgerufen werden, zu messen.

In einer weiteren bevorzugten Ausgestaltung können mindestens zwei Nachweiseinrichtungen vorgesehen sein, die an unterschiedlichen Stellen der Vorrichtung angeordnet sein können. Insbesondere kann mindestens eine Nachweiseinrichtung, bevorzugt mindestens eine zusätzliche Empfangseinrichtung oder mindestens eine Rogowskispule, derart angeordnet sein, dass sie zur Erfassung des Anregungsmagnetfeldes eingerichtet sein kann, während mindestens eine weitere Nachweiseinrichtung, bevorzugt mindestens ein Magnetfeldsensor, vorzugsweise außerhalb der Vorrichtung zur Erfassung von Störfeldern angeordnet sein kann. Andere Arten von Anordnungen mindestens zweier Nachweiseinrichtungen sind jedoch denkbar.

Die vorliegende Vorrichtung umfasst eine Steuerungseinrichtung. Der Begriff der "Steuerungseinrichtung" bezeichnet hierbei eine Einrichtung, die zur Ansteuerung mindestens einer weiteren Einrichtung und zur Bestimmung mindestens eines Wertes eingerichtet ist. Im vorliegenden Fall ist die Steuerungseinrichtung eingerichtet
o zur Ansteuerung der mindestens einen Sendeeinrichtung,
o zur Ansteuerung der mindestens einen Empfangseinrichtung,
o zur Ansteuerung der mindestens einen Nachweiseinrichtung,
o zur Bestimmung der von der mindestens einen Empfangseinrichtung aufgenommenen Antwort der magnetischen Partikel auf das Anregungsmagnetfeld, und
o zur Berücksichtigung des mindestens einen Wertes des Anregungsmagnetfeldes und der Störfelder bei der Bestimmung der Antwort der magnetischen Partikel auf das Anregungsmagnetfeld.

Zu diesem Zweck kann die Steuerungseinrichtung mindestens eine integrierte Schaltung, insbesondere eine anwendungsspezifische integrierte Schaltung (ASICs) oder eine Datenverarbeitungseinrichtung, bevorzugt einen Mikrocontroller, Mikrocomputer und/oder Computer sein oder umfassen. Mindestens eine weitere Komponente kann hierbei umfasst sein, insbesondere eine Vorverarbeitungseinrichtung und/oder eine Datenerfassungseinrichtung, eine Einrichtung zum Empfang und/oder zur Vorverarbeitung der Sensorsignale, ein AD-Wandler und/oder ein Filter. Weiterhin kann die Steuerungseinrichtung mindestens einen Datenspeicher und/oder mindestens eine Schnittstelle, insbesondere eine drahtlose Schnittstelle oder eine drahtgebundene Schnittstelle, aufweisen. Die Steuerungseinrichtung kann als eine einzige Komponente ausgestaltet sein oder mindestens zwei gesonderte Komponenten umfassen, wobei mindestens die Komponente ganz oder teilweise in ein einziges elektronisches Gerät, insbesondere eine elektronische Kommunikationseinrichtung, bevorzugt ein Smartphone, ein Tablet oder ein Laptop, integriert sein kann. Andere Arten von Steuerungseinrichtungen oder elektronischen Geräten sind jedoch denkbar.

Die Steuerungseinrichtung ist somit eingerichtet zur Ansteuerung der mindestens einen Sendeeinrichtung, der mindestens einen Empfangseinrichtung und der mindestens einen Nachweiseinrichtung. Hierzu kann die Steuerungseinrichtung vorzugsweise jeweils über eine Schnittstelle zur Sendeeinrichtung, zur Empfangseinrichtung und zur Nachweiseinrichtung verfügen, wobei die Sendeeinrichtung, die Empfangseinrichtung und die Nachweiseinrichtung jeweils über die zugehörige Schnittstelle mit der Steuerungseinrichtung kommunizieren können. Die Kommunikation kann hierbei unidirektional, insbesondere von der Steuerungseinrichtung zur Sendeeinrichtung, von der Empfangseinrichtung zur Steuerungseinrichtung und von der Nachweiseinrichtung zur Steuerungseinrichtung erfolgen. Bevorzugt ist jedoch eine bidirektionale Schnittstelle, welche Daten und/oder Instruktionen in beide Richtungen versenden kann.

Die Steuerungseinrichtung ist erfindungsgemäß weiterhin eingerichtet zur Bestimmung der von der mindestens einen Empfangseinrichtung aufgenommenen Antwort der magnetischen Partikel auf das Anregungsmagnetfeld, wobei eine Berücksichtigung des mindestens einen Wertes des Anregungsmagnetfeldes und der Störfelder bei der Bestimmung der Antwort der magnetischen Partikel auf das Anregungsmagnetfeld erfolgt. Wie oben bereits erwähnt, bezeichnet der Begriff der "Berücksichtigung" ein Vorgehen, bei welchem zur Bestimmung eines gesuchten Wertes aus einem ersten Messsignal, d.h. aus der von der mindestens einen Empfangseinrichtung aufgenommenen Antwort der magnetischen Partikel auf das Anregungsmagnetfeld, ein zweites Messsignal, d.h. der mindestens eine Wertes des Anregungsmagnetfeldes und der Störfelder, mit einfließt.

In einer bevorzugten Ausgestaltung kann die Steuerungseinrichtung dazu eingerichtet sein, dass die Berücksichtigung des mindestens einen Wertes des Anregungsmagnetfeldes und der Störfelder bei der Bestimmung der Antwort der magnetischen Partikel auf das Anregungsmagnetfeld dadurch erfolgt, dass mindestens ein erstes Messsignal in Abhängigkeit von der Antwort der magnetischen Partikel auf das Anregungsmagnetfeld und mindestens ein zweites Messsignal in Abhängigkeit von dem mindestens einen Wert des Anregungsmagnetfeldes und der Störfelder voneinander subtrahiert werden. Hierbei können vorzugsweise mindestens zwei Transferfunktionen in eine Beziehung zueinander gesetzt werden. Der Begriff der "Transferfunktion" bezeichnet eine Funktion, welche ein Verhältnis zwischen einem Ausgangssignal und einem Eingangssignal wiedergibt. Im vorliegenden Falle bezieht sich jede Transferfunktion auf ein Verhältnis zwischen dem am Ausgang der mindestens einen Empfangseinrichtung anliegenden Messsignal und einem am Eingang in die mindestens eine Empfangseinrichtung anliegenden Eingangssignal. Auf diese Weise lassen sich Eigenschaften des Messsignals und der zur Erfassung des Messsignals eingerichteten Empfangseinrichtung berücksichtigen. In einer besonderen Ausgestaltung können hierzu die Eingangssignale in einer gemeinsamen Domäne, vorzugsweise dem magnetischen Moment der magnetischen Partikel, in eine Beziehung zueinander gesetzt werden, siehe Florian Thieben et al., s.o. Hierdurch kann eine Subtraktion des mindestens einen Wertes des Anregungsmagnetfeldes und/oder der Störfelder von dem Messsignal durchgeführt werden.

In einer weiteren Ausgestaltung kann die Steuerungseinrichtung dazu eingerichtet sein, dass die Berücksichtigung des mindestens einen Wertes des Anregungsmagnetfeldes und der Störfelder bei der Bestimmung der Antwort der magnetischen Partikel auf das Anregungsmagnetfeld unter Verwendung von maschinellem Lernen erfolgt. Der Begriff des "maschinellen Lernens" bezieht sich auf ein computerimplementiertes Verfahren, bei dem eine Vorrichtung während einer "Trainingsphase" dazu eingerichtet ist, aus Eingabedaten einen Satz von Parametern zu bestimmen, der es ermöglicht, Ausgabedaten zu erzeugen, die dazu dienen, eine Vielzahl von Vorlagenätzen zu approximieren, wobei die Bestimmung in einem iterativen Prozess erfolgt, der eine Approximation an die Vielzahl der Vorlagensätze fortlaufend verbessert. Nach der Trainingsphase wird der Parametersatz zur Erzeugung von weiteren Ausgabedaten unter Verwendung weiterer Eingabedaten verwendet, wobei von den erhaltenen Ausgabedaten vernünftigerweise erwartet wird, dass sie sich den "wahren" Ausgabedaten in derselben Weise annähern, wie dies während der Trainingsphase erreicht wurde. Auf diese Weise kann während der Trainingsphase eine genauere Approximation der Antwort der magnetischen Partikel auf das Anregungsmagnetfeld mit verringertem Einfluss des mindestens einen Anregungsfeldes oder der Störfelder erzielt werden. Insbesondere kann zur Ausführung des maschinellen Lernens ein neuronales Netzwerk verwendet werden; ein anderes Vorgehen ist jedoch möglich.

In einem weiteren Aspekt betrifft die vorliegende Erfindung ein Verfahren zur Bestimmung einer Antwort von magnetischen Partikeln auf ein Anregungsmagnetfeld. Das vorliegende Verfahren umfasst hierbei zumindest die folgenden Verfahrensschritte, welche nacheinander in der angegebenen Reihenfolge oder, vorzugsweise, zumindest teilweise auch gleichzeitig ablaufen können. Die Verfahrensschritte sind zumindest:
a) Erzeugen eines Anregungsmagnetfeldes zur Anregung von magnetischen Partikeln mittels mindestens einer Sendeeinrichtung;
b) Aufnehmen einer Antwort der magnetischen Partikel auf das Anregungsmagnetfeld mittels mindestens einer Empfangseinrichtung;
c) Ansteuern der mindestens einen Sendeeinrichtung und der mindestens einen Empfangseinrichtung zum Erfassen der von der mindestens einen Empfangseinrichtung aufgenommenen Antwort der magnetischen Partikel auf das Anregungsmagnetfeld mittels einer Steuerungseinrichtung;
d) Aufnehmen mindestens eines Wertes des Anregungsmagnetfeldes oder von Störfeldern mittels mindestens einer Nachweiseinrichtung;
e) Extrahieren mindestens eines Signals aus einem von der mindestens einen Empfangseinrichtung erzeugten ersten Messsignals; und
f) Ansteuern der mindestens einen Nachweiseinrichtung und Berücksichtigen des mindestens einen Wertes des Anregungsmagnetfeldes und der Störfelder bei der Bestimmung der Antwort der magnetischen Partikel auf das Anregungsmagnetfeld mittels der Steuerungseinrichtung.

In einer besonders bevorzugten Ausgestaltung des vorliegenden Verfahrens kann für das Extrahieren des mindestens eines Signals gemäß Schritt e) zusätzlich ein von der mindestens einen Nachweiseinrichtung erzeugtes weiteres Messsignals verwendet werden.

Für weitere Einzelheiten in Bezug auf das vorliegende Verfahren wird auf die Beschreibung der Vorrichtung verwiesen.

Das vorliegende Verfahren und die beschriebene Vorrichtung eignen sich insbesondere zur tomographischen Bildgebung mittels magnetischen Gleich- und Wechselfelder, die auf magnetische Partikel, bevorzugt magnetische Nanopartikel, einwirken, die vorzugsweise verteilt in einem Fluid oder im Inneren eines Objekts, insbesondere in einem lebenden Patienten, vorliegen können. Der Begriff des "Fluids" bezeichnet hierbei eine flüssige und/oder gasförmige Phase. Hiermit kann insbesondere *in vivo* eine räumliche Verteilung der magnetischen Partikel innerhalb eines Volumens bestimmt werden.

### Vorteile der Erfindung

Die vorgeschlagene Vorrichtung und das zugehörige Verfahren weisen, im Vergleich zu aus dem Stand der Technik bekannten Vorrichtungen und Verfahren zur Bestimmung einer Antwort von magnetischen Partikeln auf ein Anregungsmagnetfeld eine Reihe von Vorteilen auf. Sie ermöglichen es insbesondere, bei der Bestimmung der Antwort der magnetischen Partikel auf ein Anregungsmagnetfeld dynamische Veränderungen in dem von der Empfangseinrichtung aufgenommenen Messsignal zu berücksichtigen, die in der Regel insbesondere in Folge von Änderungen des Anregungsmagnetfeldes aufgrund von Temperaturänderungen in der Sendekette als auch in Folge von üblicherweise vorhandenen Störfeldern, die von elektrischen Zuleitungen, aber auch von externen Quellen herrühren können, auftreten können. Hierdurch kann vor allem ein präziser Hintergrundabzug erfolgen, was ein Gewinn im Signal-zu-Hintergrund-Verhältnis bedeutet und damit eine Verbesserung des Detektionslimits der Vorrichtung und des Verfahrens. Auf diese Weise können insbesondere geringe Partikelmengen detektiert werden und/oder Vorrichtungen bereitgestellt werden, die, vor allem im Hinblick auf Anwendungen an Personen, geringere Anforderungen an die Sendekette und/oder an eine Abschirmung aufweisen können.

Hierin werden die Begriffe "haben", "aufweisen", "umfassen" oder "einschließen" oder beliebige grammatikalische Abweichungen davon in nicht-ausschließlicher Weise verwendet. Dementsprechend können sich diese Begriffe sowohl auf Situationen beziehen, in welchen, neben den durch diese Begriffe eingeführten Merkmalen, keine weiteren Merkmale vorhanden sind, oder auf Situationen, in welchen ein oder mehrere weitere Merkmale vorhanden sind. Beispielsweise kann sich der Ausdruck "A hat B", "A weist B auf", "A umfasst B" oder "A schließt B ein" sowohl auf die Situation beziehen, in welcher, abgesehen von B, kein weiteres Element in A vorhanden ist (d.h. auf eine Situation, in welcher A ausschließlich aus B besteht), als auch auf die Situation, in welcher, zusätzlich zu B, ein oder mehrere weitere Elemente in A vorhanden sind, beispielsweise Element C, Elemente C und D oder sogar weitere Elemente.

Weiterhin wird darauf hingewiesen, dass die Begriffe "mindestens ein" und "ein oder mehrere" sowie grammatikalische Abwandlungen dieser Begriffe, wenn diese in Zusammenhang mit einem oder mehreren Elementen oder Merkmalen verwendet werden und ausdrücken sollen, dass das Element oder Merkmal einfach oder mehrfach vorgesehen sein kann, in der Regel lediglich einmalig verwendet werden, beispielsweise bei der erstmaligen Einführung des Merkmals oder Elementes. Bei einer nachfolgenden erneuten Erwähnung des Merkmals oder Elementes wird der entsprechende Begriff "mindestens ein" oder "ein oder mehrere" in der Regel nicht mehr verwendet, ohne dass hierdurch die Möglichkeit eingeschränkt wird, dass das Merkmal oder Element einfach oder mehrfach vorgesehen sein kann.

Weiterhin werden hierin die Begriffe "vorzugsweise", "insbesondere", "beispielsweise" oder ähnliche Begriffe in Verbindung mit optionalen Merkmalen verwendet, ohne dass alternative Ausführungsformen hierdurch beschränkt werden. So sind Merkmale, welche durch diese Begriffe eingeleitet werden, optionale Merkmale, und es ist nicht beabsichtigt, durch diese Merkmale den Schutzumfang der Ansprüche und insbesondere der unabhängigen Ansprüche einzuschränken. So kann die Erfindung, wie der Fachmann erkennen wird, auch unter Verwendung anderer Ausgestaltungen durchgeführt werden. In ähnlicher Weise werden Merkmale, welche durch "in einer Ausführungsform der Erfindung" oder durch "in einem Ausführungsbeispiel der Erfindung" eingeleitet werden, als optionale Merkmale verstanden, ohne dass hierdurch alternative Ausgestaltungen oder der Schutzumfang der unabhängigen Ansprüche eingeschränkt werden soll. Weiterhin sollen durch diese einleitenden Ausdrücke sämtliche Möglichkeiten unangetastet bleiben, die hierdurch eingeleiteten Merkmale mit anderen Merkmalen zu kombinieren, seien es optionale oder nicht-optionale Merkmale.

### Kurze Beschreibung der Figuren

Weitere Einzelheiten und Merkmale der vorliegenden Erfindung ergeben sich aus der nachfolgenden Beschreibung von bevorzugten Ausführungsbeispielen, insbesondere in Verbindung mit den abhängigen Ansprüchen. Hierbei können die jeweiligen Merkmale für sich alleine oder zu mehreren in Kombination miteinander verwirklicht sein. Die Erfindung ist jedoch nicht auf die Ausführungsbeispiele beschränkt. Die Ausführungsbeispiele sind schematisch in den nachfolgenden Figuren dargestellt. Hierbei bezeichnen gleiche Bezugsziffern in den Figuren gleiche oder funktionsgleiche Elemente bzw. hinsichtlich ihrer Funktionen einander entsprechende Elemente.

Im Einzelnen zeigen:
- Figur 1: eine schematische Darstellung einer aus dem Stand der Technik bekannten Vorrichtung zur Bestimmung einer Antwort von magnetischen Partikeln auf ein Anregungsmagnetfeld; und
- Figuren 2 bis 6: schematische Darstellungen von bevorzugten Ausführungsbeispielen einer erfindungsgemäßen Vorrichtung zur Bestimmung einer Antwort von magnetischen Partikeln auf ein Anregungsmagnetfeld.

### Beschreibung der Ausführungsbeispiele

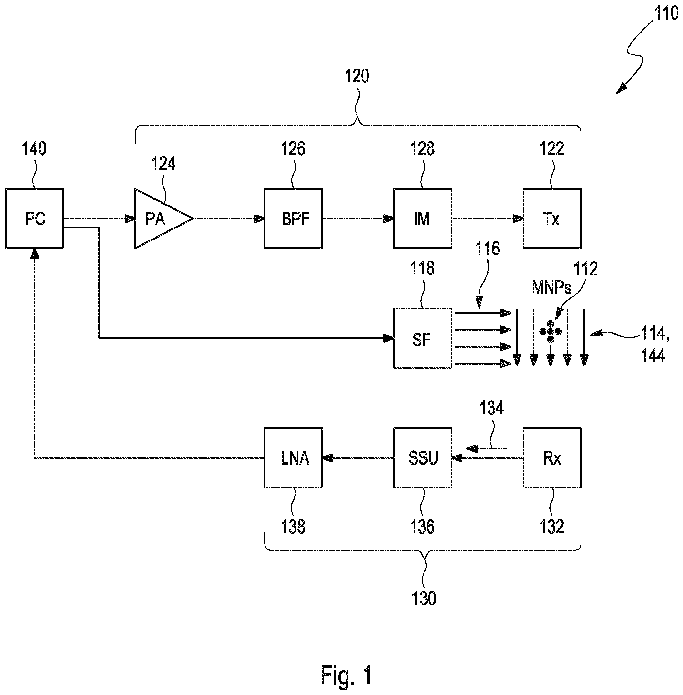

Figur 1 zeigt eine schematische Darstellung einer aus dem Stand der Technik bekannten Vorrichtung 110 zur Bestimmung einer Antwort von magnetischen Partikeln 112, bevorzugt von magnetischen Nanopartikeln *(engl.* "magnetic nanoparticles", MNPs), besonders bevorzugt von super-paramagnetischen Eisenoxid-Nanopartikel *(engl.* "superparamagnetic iron oxide nanoparticle", SPION), auf ein Anregungsmagnetfeld 114, insbesondere um eine räumliche Verteilung der magnetischen Partikel 112 *in vivo* zu bestimmen. Zusätzlich kann das die magnetischen Partikel 112 anregende Anregungsmagnetfeld 114 hierbei, wie in Figur 1 schematisch dargestellt, von einem Selektionsmagnetfeld *(engl.* "selection field", SF) 116 überlagert sein, welches mittels einer Einrichtung 118 zur Erzeugung des Selektionsmagnetfeldes 116 erzeugt wird. und das zur Eingrenzung auf ein festgelegtes Volumen eingesetzt werden kann.

Zur Erzeugung eines Anregungsmagnetfeldes 114, das zur Anregung der magnetischen Partikel 112 dient, weist die Vorrichtung 110 eine Sendekette 120 auf, welche zumindest eine Sendeeinrichtung (Tx) 122 umfasst, die vorzugsweise als Sendespule ausgeführt sein kann, die das gewünschte Anregungsmagnetfeld 114 mittels Beaufschlagung durch einen elektrischen Strom erzeugt. Die in Figur 1 schematisch dargestellte Sendekette 120 umfasst weiterhin optionale Komponenten, darunter einen Leistungsverstärker *(engl.* "power amplifier", PA) 124, einen Bandpassfilter *(engl.* "band pass filter", BPF) 126 und eine Einrichtung *(engl.* "impedance matching device", IM) 128 zur Impedanz-Anpassung, welche der Sendeeinrichtung 122 so vorgeschaltet sind, dass der die Sendeeinrichtung 122 beaufschlagende Strom zuvor den Leistungsverstärker 124, den Bandpassfilter 126 und die Einrichtung 128 zur Impedanz-Anpassung durchläuft, bevor die Sendeeinrichtung 122 damit beaufschlagt wird. Die Verstärkung des Leistungsverstärkers 124 kann bevorzugt so eingestellt sein, dass die Leistung des von der Sendeeinrichtung 122 ausgehenden elektronischen Signals ungefähr der vorgesehenen Leistung des Anregungsmagnetfelds 114 entspricht. Das Bandpassfilter 126 kann vorzugsweise derart eingerichtet sein, dass es nur diejenigen Frequenzen durchlässt, die zur Erzeugung des Anregungsmagnetfeldes 114 vorgesehen sind. Die Einrichtung 128 zur Impedanz-Anpassung dient hier zur Anpassung einer Impedanz eines Eingangs der Sendeeinrichtung 122 an die Impedanz des Leistungsverstärkers 124.

Zur Aufnahme und Weiterleitung einer Antwort der magnetischen Partikel 112 auf das Anregungsmagnetfeld 114 weist die Vorrichtung 110 weiterhin eine Empfangskette 130 auf, die zumindest eine Empfangseinrichtung (Rx) 132, die vorzugsweise als Empfangsspule ausgeführt sein kann, in welcher das Anregungsmagnetfeld 114 mittels Beaufschlagung einen elektrischen Strom induziert, der als erstes Messsignal 134 dient. Die in Figur 1 schematisch dargestellte Empfangskette 130 umfasst weitere Komponenten, darunter eine Signaltrennungseinrichtung *(engl.* "signal separation device", SSU) 136 und einen optionalen rauscharmen Verstärker *(engl.* "low-noise amplifier", LNA) 138, welche der Empfangseinrichtung 132 so nachgeschaltet sind, dass das von der Empfangseinrichtung 132 erzeugte erste Messsignal 134 anschließend die Signaltrennungseinrichtung 136 und den rauscharmen Verstärker 138 in der Empfangskette 130 durchläuft, bevor es zur Steuerungseinrichtung 140 gelangt. Die Signaltrennungseinrichtung 136 ist eingerichtet ist, um aus dem ersten Messsignal 134 ein oder mehrere Signale zu extrahieren. Hierzu kann sie bevorzugt als Bandstoppfilter ausgestaltet sein, wobei das Bandstoppfilter vorzugsweise derart ausgeführt ist, dass es diejenigen Frequenzen herausfiltert, die den Frequenzen des Anregungsmagnetfeldes 114 entsprechen. Der rauscharme Verstärker 138 verarbeitet hier ein aus dem Bandstoppfilter eingehendes elektronisches Signal so, dass ein aus dem rauscharmen Verstärker 138 ausgehenden elektronischen Signals, das an die Steuerungseinrichtung 140 weitergeleitet wird, ein möglichst hohes Signal-zu-Rausch-Verhältnis *(engl.* "signal-to-noise ratio", SNR) aufweist.

Zur Bestimmung der von der Empfangseinrichtung 132 aufgenommenen Antwort der magnetischen Partikel 112 auf das Anregungsmagnetfeld 114 weist die Vorrichtung 110 weiterhin die Steuerungseinrichtung 140 auf, die in der Ausführung gemäß Figur 1 als Computer *(engl.* "personal computer", PC) ausgeführt ist. Eine Ausführung der Steuerungseinrichtung 140 als elektronische Kommunikationseinrichtung, bevorzugt als Smartphone, Tablet oder Laptop, ist jedoch ebenfalls möglich. Die in Figur 1 schematisch dargestellte Steuerungseinrichtung 140 dient weiterhin zur Ansteuerung der Sendekette 120, insbesondere der Sendeeinrichtung 122, und der Empfangskette 130, insbesondere der Empfangseinrichtung 132. Zur Herstellung von unidirektionaler oder bevorzugt bidirektionaler Kommunikation kann die Steuerungseinrichtung 140 vorzugsweise jeweils eine Schnittstelle zur Sendekette 120, insbesondere zur Sendeeinrichtung 122, und zur Empfangskette 130, insbesondere zur Empfangseinrichtung 132, aufweisen, wobei die Sendeeinrichtung, die Empfangseinrichtung und eine Nachweiseinrichtung 142 jeweils über die zugehörige Schnittstelle mit der Steuerungseinrichtung kommunizieren können.

Die in Figur 1 schematisch dargestellte Vorrichtung 110 eignet sich insbesondere zur Erzeugung eines Anregungsmagnetfeldes 114 in einer Dimension; für die Erzeugung eines Anregungsmagnetfeldes 114 in 2 oder 3 Dimensionen können die Sendekette120 und die Empfangskette 130 in der Vorrichtung 110 in doppelter bzw. dreifacher Ausführung bereitgestellt werden.

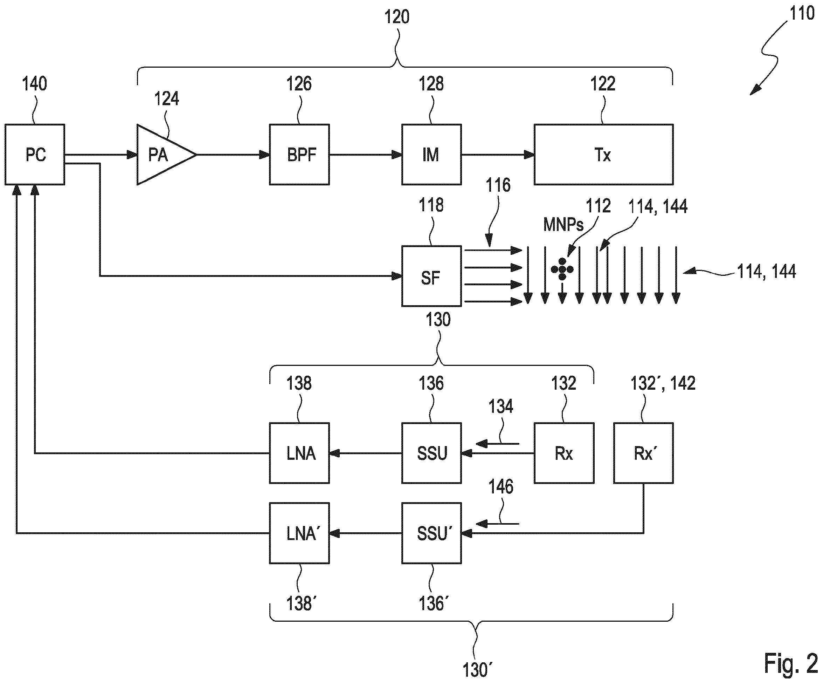

Die Figuren 2 bis 6 zeigen jeweils eine schematische Darstellung eines bevorzugten Ausführungsbeispiels einer erfindungsgemäßen Vorrichtung 110 zur Bestimmung einer Antwort von magnetischen Partikeln auf ein Anregungsmagnetfeld. Die in den Figuren 2 bis 6 schematisch dargestellten Vorrichtungen 110 umfassen jeweils dieselben notwendigen oder optionalen Komponenten gemäß der Vorrichtung aus Figur 1, zu deren Beschreibung daher auf die obige Darstellung zur Figur 1 verwiesen wird.

Die in den Figuren 2 bis 6 schematisch dargestellten Vorrichtungen 110 weisen jeweils eine Nachweiseinrichtung 142 auf, die zur Aufnahme mindestens eines Wertes des Anregungsmagnetfeldes 114 oder von Störfeldern 144 eingerichtet ist. Störfelder 144 sind ein weiteres magnetisches Feld, das ein, auch als "Hintergrundsignal" bezeichnetes weiteres Messsignal 146 in der Nachweiseinrichtung 142 erzeugen kann, wobei das Hintergrundsignal weder durch das Anregungsmagnetfeld 114 noch durch die Antwort der magnetischen Partikel 112 auf das Anregungsmagnetfeld 114 erzeugt wird. Als Quellen für die Störfelder 144 kommen einerseits elektrische Komponenten und Leitungen innerhalb der Vorrichtung 110 und andererseits externe Quellen außerhalb der Vorrichtung 110, insbesondere von elektrischen Komponenten und Leitungen, welche sich in der Nähe der Vorrichtung 110 befinden, in Frage. Die Nachweiseinrichtung 142 kann somit insbesondere dazu dienen, um zeitliche Änderungen des Anregungsmagnetfeldes aufgrund von Temperaturänderungen in der Sendekette 120, die insbesondere in Folge einer Erwärmung von elektrischen Komponenten und Leitungen in der Sendekette 120, typischerweise in der Sendeeinrichtung 122 und dem Leistungsverstärker 124, auftreten können, und/oder um ein Vorhandensein von Störfeldern 144 nachzuweisen. Im Unterschied hierzu verfügt die aus dem Stand der Technik bekannte Vorrichtung 110 gemäß Figur 1 über keine Nachweiseinrichtung 142, so dass dort auftretende Störfelder 144 weder erfasst noch berücksichtigt werden können.

Wie die Figuren 2 bis 4 und 6 schematisch zeigen, kann die Nachweiseinrichtung 142 als weitere Empfangskette 130' ausgeführt sein und insbesondere eine weitere Signaltrennungseinrichtung (SSU') 136' und einen weiteren rauscharmen Verstärker (LNA') 138', welche der Nachweiseinrichtung 142 so nachgeschaltet sind, dass das von der Nachweiseinrichtung 142 erzeugte weiteres Messsignal 146 anschließend die weitere Signaltrennungseinrichtung 136' und den weitere rauscharmen Verstärker 138' in der Empfangskette 130 durchläuft, bevor es ebenfalls zur Steuerungseinrichtung 140 gelangt. Die Steuerungseinrichtung 140 ist hierbei ferner zur Ansteuerung der Nachweiseinrichtung 142 und zur Berücksichtigung des mindestens einen Wertes des Anregungsmagnetfeldes 114 und der Störfelder 144 bei der Bestimmung der Antwort der magnetischen Partikel 112 auf das Anregungsmagnetfeld eingerichtet. Vorzugsweise kann hierbei die Aufnahme des ersten Messsignals 134, welches der Antwort der magnetischen Partikel 112 auf das Anregungsmagnetfeld 114 entspricht, im gleichen Zeitraum erfolgen wie die Aufnahme des weiteres Messsignals 146, welches der Summe des mindestens einen Wertes des Anregungsmagnetfeldes 114 und der Störfelder 144 entspricht. Diese Ausführung weist den Vorteil auf, dass sich das erste Messsignal 134 und das weiteres Messsignal 146, die beide zur Bestimmung der Antwort der magnetischen Partikel 112 auf das Anregungsmagnetfeld 114 einfließen, sich auf denselben Zeitraum beziehen. Auf diese Weise lassen sich zeitliche Veränderungen in dem ersten Messsignal 134 berücksichtigen, die einerseits auf dynamische Veränderungen in dem Anregungsmagnetfeld 116 und/oder in der Antwort der magnetischen Partikel 112 auf das Anregungsmagnetfeld 114 zurückgehen oder andererseits auf Störfelder 114 innerhalb und/oder außerhalb der Vorrichtung 110 beruhen.

In der schematischen Darstellung gemäß Figur 2 ist die Nachweiseinrichtung 142 als zusätzliche Empfangseinrichtung (Rx') 132' ausgeführt, die zusätzlich zur Empfangseinrichtung (Rx) 132 an die Steuerungseinrichtung 140 geführt wird. Bevorzugt kann die zusätzliche Empfangseinrichtung 132' eine Empfangsspule aufweisen, vorzugsweise ein weiteres Exemplar der von der Empfangseinrichtung 132 umfassten Empfangsspule. Während die von der Empfangseinrichtung 132 umfasste Empfangsspule zur Bestimmung der Antwort der magnetischen Partikel 112 auf das Anregungsmagnetfeld 114 eingerichtet ist, ist die zusätzliche Empfangsspule 132' bevorzugt derart angeordnet, dass hierdurch der mindestens eine Wert für das Anregungsmagnetfeld 114 und/oder die Störfelder 144, nicht jedoch die Antwort der magnetischen Partikel 112 auf das Anregungsmagnetfeld 114 erfasst werden kann.

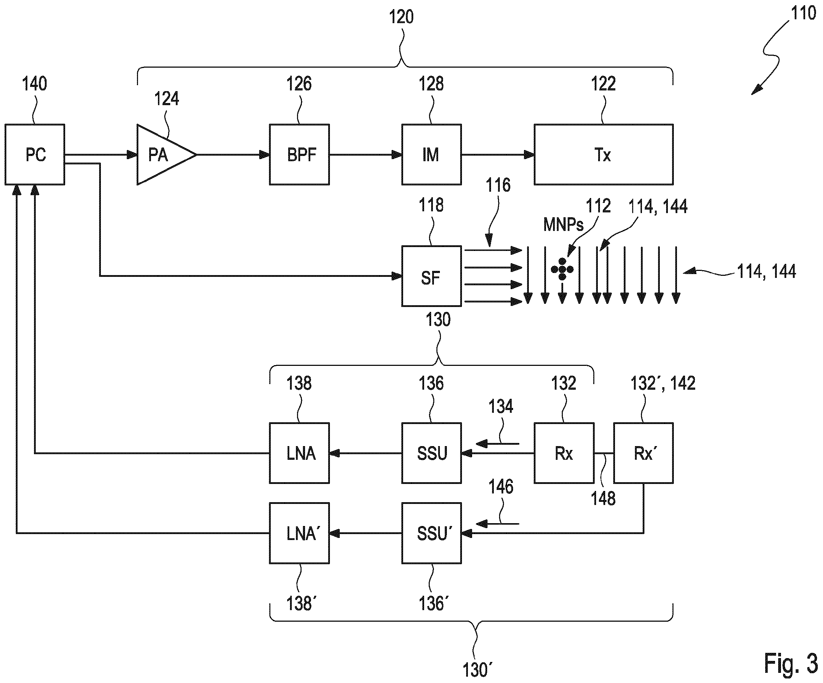

In der schematischen Darstellung gemäß Figur 3 ist die Nachweiseinrichtung 142 ebenfalls als zusätzliche Empfangseinrichtung (Rx') 132' ausgeführt. In dieser Ausführung ist die zusätzliche Empfangseinrichtung 132' jedoch derart mit der Empfangseinrichtung 132 in Serie verschaltet, dass eine destruktive Überlagerung 148 auftritt, wodurch sich die von der jeweiligen Einrichtung 132, 132' erzeugten Messsignale 134, 146 dann gegenseitig auslöschen, wenn keine magnetischen Partikel 112 in dem Volumen, das von dem Anregungsmagnetfeld 114 beaufschlagt wird, vorhanden sind. Wird die zusätzliche Empfangseinrichtung 132' derart angeordnet, dass zusätzlich zu dem Anregungsmagnetfeld 114 und der Antwort der magnetischen Partikel 112 auf das Anregungsmagnetfeld 114 zusätzlich auch noch die Störfelder 144 zum weiteren Messsignal 146 beitragen können, so kann die zusätzliche Empfangseinrichtung 132' in dieser Ausgestaltung das Hintergrundsignal, das von den Störfeldern 144 erzeugt wird, direkt als das weitere Messsignal 146 bereitstellen.

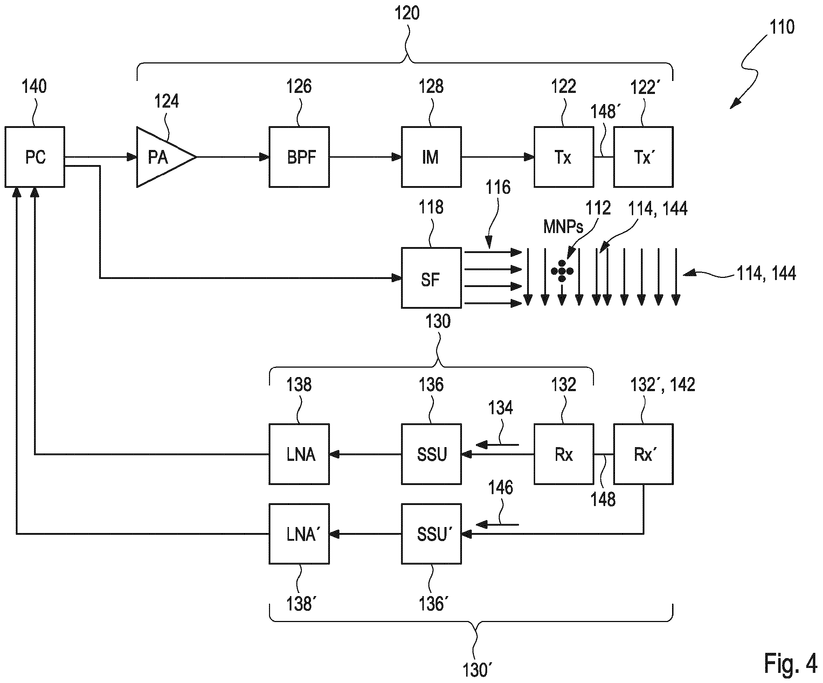

In der Ausführung gemäß Figur 4 weist die Vorrichtung 110 zusätzlich zu der Darstellung in Figur 3 eine zusätzliche Sendeeinrichtung (Tx') 122' auf, die ebenfalls in Serie mit der Sendeeinrichtung 122, die zur Erzeugung des Anregungsmagnetfeldes 114 zur Anregung der magnetischen Partikel 112 eingerichtet ist, geschaltet sein. Bevorzugt kann auch hier die zusätzliche Sendeeinrichtung 122' eine Sendespule aufweisen, vorzugsweise ein weiteres Exemplar der von der Sendeeinrichtung 122 umfassten Sendespule. Vorzugsweise kann die zusätzliche Sendespule 122' derart angeordnet sein, dass hierdurch ein Magnetfeld mit denselben Eigenschaften wie das Anregungsmagnetfeld 116 erzeugt wird, jedoch keine Anregung der magnetischen Partikel 112 erfolgen kann. In der Ausführung gemäß Figur 4 kann die zusätzliche Sendeeinrichtung 122' derart mit der mindestens einen Sendeeinrichtung 122' verschaltet sein, dass sich hierdurch eine Auslöschungseinrichtung ergibt, in welcher ebenfalls eine destruktive Überlagerung 148' der beiden Sendeeinrichtungen 122, 122' erfolgt. Da hier die zusätzliche Empfangseinrichtung 132' analog zur Darstellung in Figur 3 derart angeordnet ist, dass zusätzlich zu dem Anregungsmagnetfeld 114 und der Antwort der magnetischen Partikel 112 auf das Anregungsmagnetfeld 114 zusätzlich noch die Störfelder 144 zum weiteren Messsignal 146 beitragen können, so kann die zusätzliche Empfangseinrichtung 132' in dieser Ausgestaltung das Hintergrundsignal, das von den Störfeldern 144 erzeugt wird, direkt als das weitere Messsignal 146 bereitstellen.

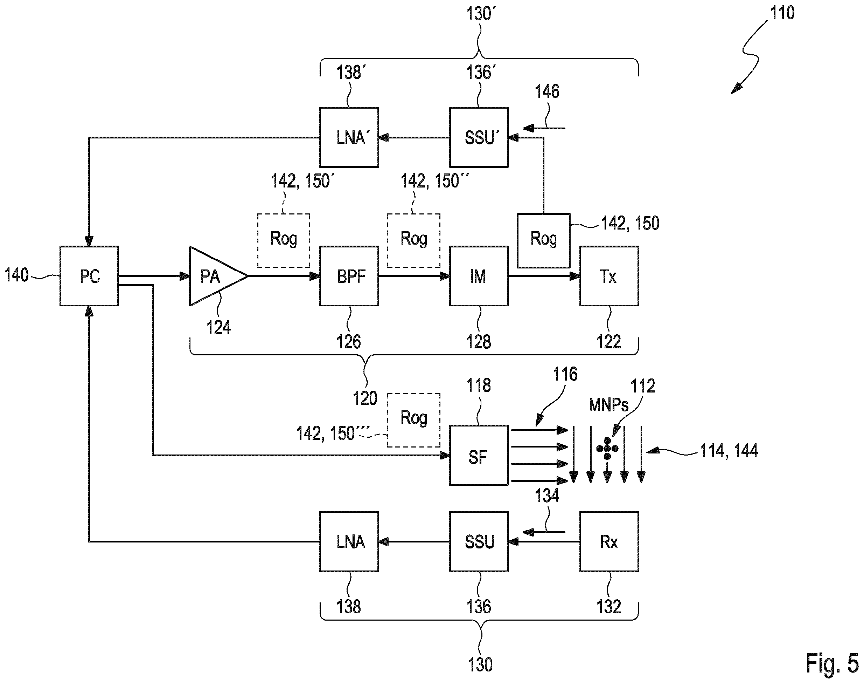

In der schematischen Darstellung gemäß Figur 5 ist die Nachweiseinrichtung 142 als Einrichtung zum Nachweis eines elektrischen Stroms, insbesondere als Rogowskispule 150, ausgeführt, die hier in bevorzugt Weise in unmittelbarer Nähe der mindestens einen Sendeeinrichtung 122, besonders bevorzugt zwischen der Einrichtung 128 zur Impedanz-Anpassung und der mindestens einen Sendeeinrichtung 122, angeordnet ist. Auf diese Weise kann ein Strom in der Sendekette 120 erfasst werden, der sich zur Bestimmung des mindestens einen Wertes des Anregungsmagnetfelds 114 eignet. Alternativ oder zusätzlich können ein oder mehrere Rogowskispulen 150, 150', 150", 150‴ an einer beliebigen Stelle in der mindestens einen Sendekette 120 angeordnet sein.

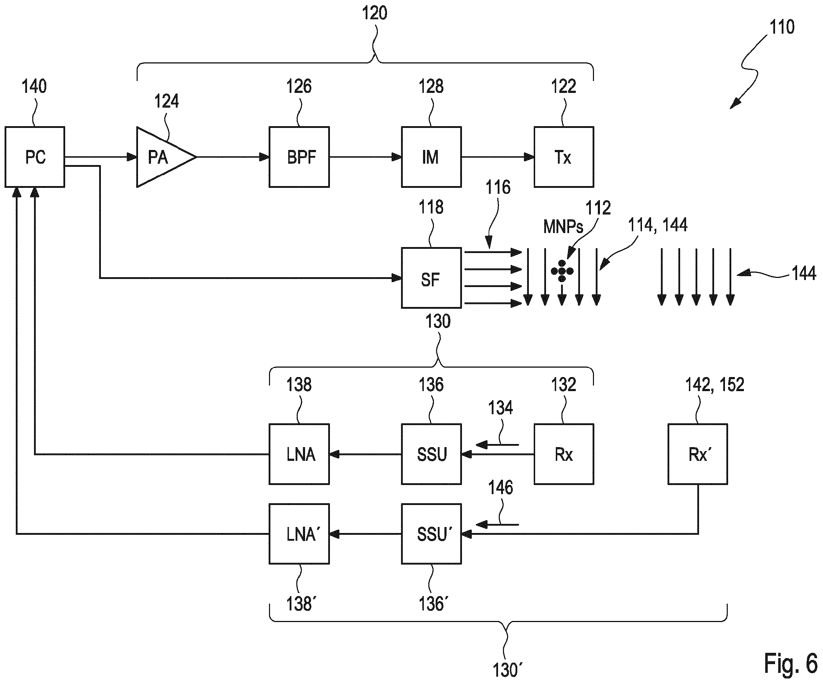

In der schematischen Darstellung gemäß Figur 6 ist die Nachweiseinrichtung 142 als Magnetfeldsensor 152 ausgeführt. Der Magnetfeldsensor 152 kann vorzugsweise eine Hall-Sonde oder einen XMR-Sensor umfassen, die sich aufgrund ihres kompakten Aufbaus besonders für den vorliegenden Zweck eignen; die Verwendung eines anderen Magnetfeldsensors ist jedoch möglich. Wie Figur 6 zeigt, kann der Magnetfeldsensor 152 in bevorzugter Weise außerhalb des Anregungsmagnetfeldes 114 angeordnet sein, insbesondere um auf diese Weise Störfelder 144, die von externen Quellen außerhalb der Vorrichtung 110, insbesondere von elektrischen Komponenten und Leitungen, welche sich in der Nähe der Vorrichtung 110 befinden, hervorgerufen werden, als das weitere Messsignal 146 zu erfassen. Alternativ oder zusätzlich (nicht dargestellt), kann der Magnetfeldsensor 152 innerhalb des Anregungsmagnetfeldes 114, jedoch außerhalb des Volumens angeordnet sein, in welchem die magnetischen Partikel 112 vorliegen.

In weiteren Ausführungen (nicht dargestellt) kann die Vorrichtung 100 mindestens zwei Nachweiseinrichtungen 142 umfassen, die an unterschiedlichen Stellen der Vorrichtung 110 angeordnet sein können. Insbesondere kann eine der Nachweiseinrichtungen 142, bevorzugt die zusätzliche Empfangseinrichtung 132' oder die Einrichtung zum Nachweis eines elektrischen Stroms, insbesondere die Rogowskispule 150, derart angeordnet sein, dass sie zur Erfassung des Anregungsmagnetfeldes 114 eingerichtet ist, während eine weitere Nachweiseinrichtung 142, bevorzugt der Magnetfeldsensor 152, vorzugsweise außerhalb der Vorrichtung 110 zur Erfassung der Störfelder 144 angeordnet sein kann. Andere Arten von Anordnungen mindestens zweier Nachweiseinrichtungen sind jedoch denkbar.

### Bezugszeichenliste

- 110: Vorrichtung zur Bestimmung einer Antwort von magnetischen Partikeln auf ein Anregungsmagnetfeld
- 112: magnetische Partikel
- 114: Anregungsmagnetfeld
- 116: Selektionsmagnetfeld
- 118: Einrichtung zur Erzeugung eines Selektionsmagnetfeldes
- 120: Sendekette
- 122, 122': Sendeeinrichtung
- 124: Leistungsverstärker
- 126: Bandpassfilter
- 128: Einrichtung zur Impedanz-Anpassung
- 130, 130': Empfangskette
- 132, 132': Empfangseinrichtung
- 134: erstes Messsignal
- 136, 136': Signaltrennungseinrichtung
- 138, 138': rauscharmer Verstärker
- 140: Steuerungseinrichtung
- 142: Nachweiseinrichtung
- 144: Störfelder
- 146: weiteres Messsignal
- 148, 148': destruktive Überlagerung
- 150, 150', ...: Rogowskispule
- 152: Magnetfeldsensor

## Patentansprüche

1. Vorrichtung (110) zur Bestimmung einer Antwort von magnetischen Partikeln (112) auf ein Anregungsmagnetfeld (114), umfassend
- mindestens eine Sendeeinrichtung (122), die zur Erzeugung eines Anregungsmagnetfeldes (114) zur Anregung von magnetischen Partikeln (114) eingerichtet ist;
- mindestens eine Empfangseinrichtung (132), die zur Aufnahme einer Antwort der magnetischen Partikel (112) auf das Anregungsmagnetfeld (114) eingerichtet ist;
- mindestens eine Nachweiseinrichtung (142), die zur Aufnahme mindestens eines Wertes des Anregungsmagnetfeldes (114) oder von Störfeldern (144) eingerichtet ist;
- mindestens eine Signaltrennungseinrichtung (136, 136'), die dazu eingerichtet ist, um aus einem von der mindestens einen Empfangseinrichtung (132) erzeugten ersten Messsignal (134) mindestens ein Signal zu extrahieren; und
- eine Steuerungseinrichtung (140), die
o zur Ansteuerung der mindestens einen Sendeeinrichtung (122),
o zur Ansteuerung der mindestens einen Empfangseinrichtung (132),
o zur Ansteuerung der mindestens einen Nachweiseinrichtung (142),
o zur Bestimmung der von der mindestens einen Empfangseinrichtung (132) aufgenommenen Antwort der magnetischen Partikel (112) auf das Anregungsmagnetfeld (114) eingerichtet ist, und
o zur Berücksichtigung des mindestens einen Wertes des Anregungsmagnetfeldes (114) oder der Störfelder (144) bei der Bestimmung der Antwort der magnetischen Partikel (112) auf das Anregungsmagnetfeld (114) eingerichtet ist.

2. Vorrichtung (110) nach dem vorangehenden Anspruch, wobei die mindestens eine Nachweiseinrichtung (142) dazu eingerichtet ist, dass die Aufnahme des mindestens einen Wertes des Anregungsmagnetfeldes (114) oder der Störfelder (144) im gleichen Zeitraum erfolgt wie die Aufnahme der Antwort der magnetischen Partikel (112) auf das Anregungsmagnetfeld (114).

3. Vorrichtung (110) nach einem der vorangehenden Ansprüche, wobei die mindestens eine Nachweiseinrichtung (142) umfasst:
- mindestens eine zusätzliche Empfangseinrichtung (132`), die zumindest eine Empfangsspule aufweist, die derart angeordnet ist, dass hierdurch keine Bestimmung der Antwort der magnetischen Partikel (112) auf das Anregungsmagnetfeld (114) erfolgt;
- mindestens eine Einrichtung zum Nachweis eines elektrischen Stroms, insbesondere eine Rogowskispule (150); oder
- mindestens einen Magnetfeldsensor (152), der außerhalb des Anregungsmagnetfeldes (114) angeordnet ist.

4. Vorrichtung (110) nach dem vorangehenden Anspruch, wobei die mindestens eine zusätzliche Empfangseinrichtung (132') derart angeordnet ist, dass eine destruktive Überlagerung (148) zu der mindestens einen Empfangseinrichtung (132) erfolgt.

5. Vorrichtung (110) nach einem der beiden vorangehenden Ansprüche, wobei die Vorrichtung (110) ferner mindestens eine zusätzliche Sendeeinrichtung (122') umfasst, wobei die mindestens eine zusätzliche Sendeeinrichtung (122') in Serie mit der mindestens einen Sendeeinrichtung (122) geschaltet ist.

6. Vorrichtung (110) nach einem der vorangehenden Ansprüche, wobei die mindestens eine Signaltrennungseinrichtung (136, 136') der mindestens einen Empfangseinrichtung (132) derart nachgeschaltet ist, dass das von der mindestens eine Empfangseinrichtung (132) erzeugte erste Messsignal (134) oder das von der mindestens einen Nachweiseinrichtung (142) erzeugte weitere Messsignal (146) anschließend die mindestens eine Signaltrennungseinrichtung (136, 136') durchläuft, bevor es zur Steuerungseinrichtung (140) gelangt.

7. Vorrichtung (110) nach dem vorangehenden Anspruch, wobei die mindestens eine Signaltrennungseinrichtung (136, 136') als Bandstoppfilter ausgestaltet ist, der dazu eingerichtet ist, Frequenzen aus einem festlegbaren Frequenzbereich einer hohen Dämpfung zu unterwerfen, während außerhalb des Bandes liegende Frequenzen durchgelassen werden.

8. Vorrichtung (110) nach dem vorangehenden Anspruch, wobei das Bandstoppfilter derart ausgeführt ist, dass es Frequenzen herausfiltert, die den Frequenzen des Anregungsmagnetfeldes (114) entsprechen.

9. Vorrichtung (110) nach einem der vorangehenden Ansprüche, ferner umfassend mindestens einen rauscharmen Verstärker (138, 138`), welcher der mindestens einen Empfangseinrichtung (132) derart nachgeschaltet ist, dass das von der mindestens eine Empfangseinrichtung (132) erzeugte erste Messsignal (134) oder das von der mindestens einen Nachweiseinrichtung (142) erzeugte weitere Messsignal (146) anschließend den mindestens einen rauscharmen Verstärker (138, 138') durchläuft, bevor es zur Steuerungseinrichtung (140) gelangt.

10. Vorrichtung (110) nach dem vorangehenden Anspruch, wobei der mindestens eine rauscharme Verstärker (138, 138') ein aus dem Bandstoppfilter eingehendes elektronisches Signal so verarbeitet, dass ein aus dem mindestens einen rauscharmen Verstärker (138, 138') ausgehendes elektronischen Signal, das an die Steuerungseinrichtung (140) weitergeleitet wird, ein hohes Signal-zu-Rausch-Verhältnis aufweist.

11. Vorrichtung (110) nach einem der vorangehenden Ansprüche, wobei die Steuerungseinrichtung (140) dazu eingerichtet ist, dass die Berücksichtigung des mindestens einen Wertes des Anregungsmagnetfeldes (114) oder der Störfelder (144) bei der Bestimmung der Antwort der magnetischen Partikel (112) auf das Anregungsmagnetfeld (114) dadurch erfolgt, dass das mindestens eine erste Messsignal (134) in Abhängigkeit von der Antwort der magnetischen Partikel (112) auf das Anregungsmagnetfeld (114) und das mindestens eine weitere Messsignal (146) in Abhängigkeit von dem mindestens einen Wert des Anregungsmagnetfeldes (114) oder der Störfelder (144) voneinander subtrahiert werden.

12. Vorrichtung (110) nach dem vorangehenden Anspruch, wobei die Steuerungseinrichtung (140) ferner dazu eingerichtet ist, dass mindestens zwei Transferfunktionen in eine Beziehung zueinander gesetzt werden, wobei eine erste Transferfunktion ein Verhältnis zwischen dem mindestens einen ersten Messsignal (134) und einem an der mindestens einen Empfangseinrichtung (132) anliegenden ersten Eingangssignal angibt, und wobei mindestens eine weitere Transferfunktion ein Verhältnis zwischen mindestens einem weiteren Messsignal (146) und einem an der mindestens einen zusätzlichen Empfangseinrichtung (132') anliegenden mindestens einen weiteren Eingangssignal angibt.

13. Vorrichtung (110) nach dem vorangehenden Anspruch, wobei die Steuerungseinrichtung (140) ferner dazu eingerichtet ist, dass die Eingangssignale in einer gemeinsamen Domäne, insbesondere einem magnetischen Moment der magnetischen Partikel (112), derart in eine Beziehung zueinander gesetzt werden, dass eine Subtraktion des mindestens einen Wertes des Anregungsmagnetfeldes (114) oder der Störfelder (144) von dem Messsignal durchgeführt wird.

14. Vorrichtung (110) nach einem der vorangehenden Ansprüche, ferner umfassend eine Einrichtung (118) zur Erzeugung eines mit dem die magnetischen Partikel (112) anregenden Magnetfeld (114) überlagerten Selektionsmagnetfeldes (116).

15. Verfahren zur Bestimmung einer Antwort von magnetischen Partikeln (112) auf ein Anregungsmagnetfeld (114), umfassend die Verfahrensschritte:
a) Erzeugen eines Anregungsmagnetfeldes (114) zur Anregung von magnetischen Partikeln (112) mittels mindestens einer Sendeeinrichtung (122);
b) Aufnehmen einer Antwort der magnetischen Partikel (112) auf das Anregungsmagnetfeld (114) mittels mindestens einer Empfangseinrichtung (132); und
c) Ansteuern der mindestens einen Sendeeinrichtung (122) und der mindestens einen Empfangseinrichtung (132) zum Erfassen der von der mindestens einen Empfangseinrichtung (132) aufgenommenen Antwort der magnetischen Partikel (112) auf das Anregungsmagnetfeld (114) mittels einer Steuerungseinrichtung (140),
d) Aufnehmen mindestens eines Wertes des Anregungsmagnetfeldes (114) oder von Störfeldern (144) mittels mindestens einer Nachweiseinrichtung (142);
e) Extrahieren mindestens eines Signals aus einem von der mindestens einen Empfangseinrichtung (132) erzeugten ersten Messsignals (134); und
f) Ansteuern der mindestens einen Nachweiseinrichtung (142) und Berücksichtigen des mindestens einen Wertes des Anregungsmagnetfeldes (114) oder der Störfelder (144) bei der Bestimmung der Antwort der magnetischen Partikel (112) auf das Anregungsmagnetfeld (114) mittels der Steuerungseinrichtung (140).
